(19) Europäisches Patentamt
European Patent Office
Office européen des brevets

(11) Publication number: 0 239 728
A2

(12) # EUROPEAN PATENT APPLICATION

(21) Application number: 87100699.5

(22) Date of filing: 22.06.84

(51) Int.Cl.[3]: C 07 D 237/18

(30) Priority: 23.06.83 JP 113409/83
29.07.83 JP 138878/83

(43) Date of publication of application:
07.10.87 Bulletin 87/41

(84) Designated Contracting States:
AT BE CH DE FR GB IT LI LU NL SE

(60) Publication number of the earlier application in accordance with Art. 76 EPC: 0 134 439

(71) Applicant: NISSAN CHEMICAL INDUSTRIES LTD.
3-7-1, Kanda Nishiki-cho
Chiyoda-ku Tokyo(JP)

(72) Inventor: Masakazu, Taniguchi
No. 104, 6-12, 2-chome Narashino
Funabashi-shi Chiba-ken(JP)

(72) Inventor: Masatoshi, Baba
5-32-9, Yatsu
Narashino-shi Chiba-ken(JP)

(72) Inventor: Yoshinori, Ochiai
8-9, Higashi 4-chome
Hasuda-shi Saitama-ken(JP)

(72) Inventor: Masayoshi, Hirose
501, 19-28, Kamiaoki 1-chome
Kawaguchi-shi Saitama-ken(JP)

(72) Inventor: Kiminori, Hirata
14-3, Ryoke 1-chome
Urawa-shi Saitama-ken(JP)

(74) Representative: Schön, Alfred, Dr. et al,
Patentanwälte Müller-Boré, Deufel, Schön, Hertel
Lewald, Otto Isartorplatz 6 Postfach 26 02 47
D-8000 München 26(DE)

(54) **New pyridazinone derivatives and the preparation thereof.**

(57) Novel 3(2H)-pyridazinone derivatives which, in their free acid form, are 4-halogenated-5-mercapto-3(2H)-pyridazinones having a straight or branched $C_2$ to $C_6$ alkyl substituent in the 2-position of their structure. These products are made by interaction of a corresponding 4,5-di-halogenated-3(2H)-pyridazinone with a hydrosulfide, preferably in aqueous solution. The direct product of this interaction is usually a salt, from which the free acid form can be obtained by acidification. The products are useful as intermediates for the production of insecticidal, acaricidal, nematicidal and fungicidal products for agricultural and horticultural use. Preferred compounds are 2-tertiary-butyl-4-chloro-5-mercapto-3(2H)-pyridazinone and 3-tertiary-butyl-4-bromo-5-mercapto-3(2H)-pyridazinone.



Croydon Printing Company Ltd.

# NEW PYRIDAZINONE DERIVATIVES AND THE PREPARATION THEREOF.

## BACKGROUND OF THE INVENTION.

(1) The Field of the Invention.

This invention relates to novel 3(2H)-pyridazinone derivatives and the preparation thereof. These products are useful as intermediates for the production of other 3(2H)-pyridazinone derivatives which are valuable as active ingredients for insecticidal, acaricidal, nematicidal and fungicidal compositions for use for agricultural and horticultural purposes. These active ingredients are described and claimed in our European Patent Application Number 84107189.7 (Publication Number 0134439), of which this present application is a divisional application.

(2) The Description of the prior art.

In our European Patent Application Number 84107189.7 (Publication Number 0134439), there are described and claimed novel 3(2H)-pyridazinone derivatives having the general formula:

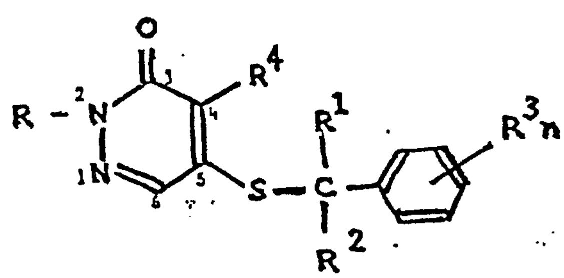

wherein

R denotes a straight or branched $C_2$ to $C_6$ alkyl;

$R^1$ and $R^2$ denote each independently hydrogen or a straight or branched $C_1$ to $C_6$ alkyl;

$R^4$ denotes a halogen;

$R^3$ denotes a halogen; a straight or branched $C_1$ to $C_{12}$ alkyl; a $C_3$ to $C_6$ cycloalkyl unsubstituted or substituted by a straight or branched $C_1$ to $C_6$ alkyl; a straight or branched $C_1$ to $C_{12}$ alkoxy; a straight or branched $C_1$ to $C_6$ haloalkyl; a straight or branched $C_1$ to $C_6$ haloalkoxy; $-CN$; $-NO_2$;

a substituent of the structure:

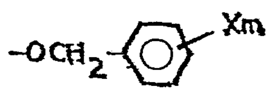 ; 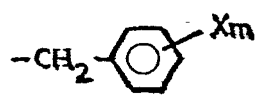 ; 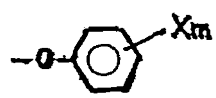 ; 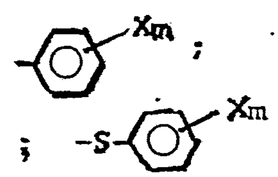 ;

(wherein X denotes a halogen, a straight or branched $C_1$ to $C_6$ alkyl, a $C_3$ to $C_6$ cycloalkyl, a straight or branched $C_1$ to $C_6$ alkoxy, a straight or branched $C_1$ to $C_6$ haloalkyl, a straight or branched $C_1$ to $C_6$ haloalkoxy, $-CN$ or $-NO_2$, and m denotes 0 or an integer of 1 to 5, said X being the same or different when m is an integer of 2 to 5);

a pyridyloxy which may be substituted by a halogen and/or $-CF_3$;

a quinoxalyloxy which may be substituted by a halogen and/or $-CF_3$;

a straight or branched $C_1$ to $C_6$ alkenyloxy;

a straight or branched $C_1$ to $C_6$ alkylthio;

a straight or branched $C_1$ to $C_6$ haloalkylthio;

$-Si(CH_3)_3$; -OH; $-N(CH_3)_2$; -SCN; $-COOCH_3$; or

$-OCH(CH_3)COOC_2H_5$;

and

n denotes an integer of 1 to 5;

said $R^3$ being the same or different when n is an integer of 2 to 5.

In the said specification there are also described processes for preparing such 3(2H)-pyridazinone derivatives, one of which comprises reacting a compound of the general formula:-

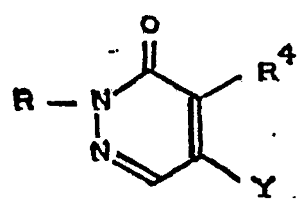

wherein R and $R^4$ have the same meanings as defined above and Y denotes -SH, with a compound of the general formula:-

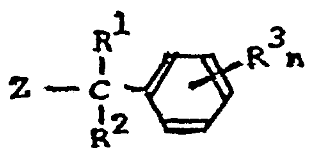

wherein $R^1$, $R^2$, $R^3$ and n have the same meanings as defined above and Z denotes a halogen.

## SUMMARY OF THE INVENTION.

An object of the invention is to provide novel 3(2H)-pyridazinone derivatives which are useful as intermediates for the manufacture of other 3(2H)-pyridazinone derivatives as described and claimed in our European Patent Application Number 84107189.7 (Publication Number 0134439), by the said process referred to therein.

Another object of the invention is to provide a process for the manufacture of the said novel 3(2H)-pyridazinone derivatives.

## DESCRIPTION OF THE PREFERRED EMBODIMENTS.

The compounds of the present invention are, in their free acid form, represented by the formula:-

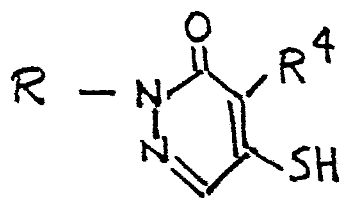

According to the invention, they may be made by a process which comprises interacting a compound of the formula:-

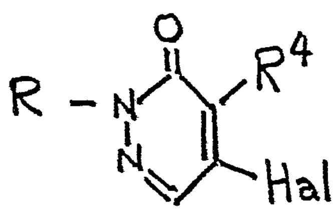

(wherein R denotes a straight or branched $C_2$ to $C_6$ alkyl; $R^4$ denotes a halogen, and Hal denotes a halogen) with a hydrosulfide.

In this reaction, the halogen in the 5-position in the pyridazinone structure is replaced by a -SH group.

The reaction may be carried out in solution, especially in aqueous solution, at elevated temperature. The preferred temperature is in the range 40 to 100 degrees C.

The hydrosulfide is most conveniently a soluble hydrosulfide, and preferably sodium hydrosulfide.

The proportions of the reactants used may be, most conveniently, a stoichiometric excess of the hydrosulfide.

As the immediate product of this reaction is thus obtained in the form of a salt, the form containing the free -SH group may be obtained from this by acidification. This is done very conveniently by adding an acid, for example hydrochloric acid, to the solution after cooling it and removing any undesired insoluble material by filtration.

The acidification should preferably be carried out to bring the solution to a pH of 2 or less.

The substituent R is preferably a straight or branched alkyl of 2 to 4 carbon atoms, such as ethyl, n-propyl, isopropyl, n-butyl, iso-butyl, sec.-butyl, or tert.-butyl, and most preferebly tert. butyl.

The substituent $R^4$ is preferably chlorine or bromine, more preferably chlorine.

The conversion of the products of the present invention into compounds having insecticidal, acaricidal, nematicidal

and fungicidal properties for agricultural and horticultural purposes may be carried out in the manner more fully described in European Patent Application Number 84107189.7 (Publication Number 0134439).

The invention is illustrated, but not limited, by the following Examples.

Example 1.

Synthesis of 2-Tert.-Butyl-4-Chloro-5-Mercapto-3(2H)-Pyridazinone.

To 560 ml. of water were added 66.3g of 2-tert.-butyl-4,5-dichloro-3(2H)-pyridazinone and 48.0g of 70% sodium hydrosulfide. After stirring at 60 degrees C for 4 hours, activated carbon was added thereto. The resulting mixture was allowed to cool and then filtered. Concentrated hydrochloric acid was added to the resulting filtrate until the pH thereof was lowered to 2 or less. The resulting solid was filtered off, washed with water, dried and then recrystallized from a mixed solvent of benzene and n-hexane to give the intended product as white needle-like crystals of m.p. 112 - 113 degrees C. (Yield: 81.5%).

The compound thus obtained was analyzed by means of $^1H$-NMR spectrum in deutero-chloroform ($CDCl_3$) to obtain the following results:-

$^1H$-NMR ($CDCl_3$), $\delta$ (ppm):

1.61 (9H,s,2-t-Bu), 4.04 (1H,s,-SH),

7.56 (1H,s,6-H).

Example 2.

Synthesis of 2-Tert.-Butyl-4-Bromo-5-Mercapto-3(2H)-Pyridazinone.

To 200 ml. of water were added 31.0g of 2-tert.-butyl-4,5-dibromo-3(2H)-pyridazinone and 15.8g of 70% sodium hydrosulfide. After stirring at 60 degrees C for 4 hours, the resulting mixture was allowed to cool to room temperature and incorporated with about 8 ml of concentrated hydrochloric acid to lower the pH to not higher than 2. The resulting solid was filtered off, washed with water, dried and then recrystallized from benzene/n-hexane to give 8.0g of the intended product as white crystals of m.p. 107-110 degrees C. (Yield: 30.48%).

The compound thus obtained was analyzed by means of $^1H$-NMR spectrum in deutero-chloroform ($CDCl_3$) to obtain the following results:-

$^1H$-NMR ($CDCl_3$), $\delta$ (ppm):

1.63 (9H,s,2-t-Bu), 4.18 (1H,s,-SH),

7.53 (1H,s,6-H).

WHAT IS CLAIMED IS:-

1. A 3(2H)-pyridazinone derivative having, in its free acid form, the general formula:-

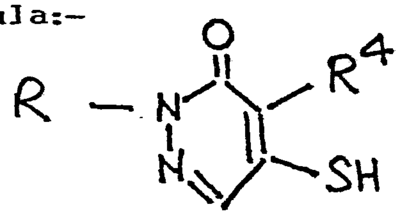

wherein R denotes a straight or branched $C_2$ to $C_6$ alkyl and $R^4$ denotes a halogen.

2. A 3(2H)-pyridazinone derivative as claimed in Claim 1, wherein $R^4$ represents chlorine or bromine.

3. A 3(2H)-pyridazinone derivative as claimed in Claim 1 or Claim 2, wherein R represents a tertiary butyl group.

4. 2-Tert.-butyl-4-chloro-5-mercapto-3(2H)-pyridazinone.

5. 2-Tert.-butyl-4-bromo-5-mercapto-3(2H)-pyridazinone.

6. A process for the production of a 3(2H)-pyridazinone derivative as claimed in any of Claims 1 to 5, which comprises interacting a compound of the formula:-

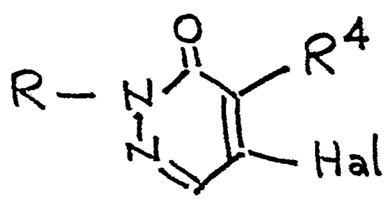

(wherein R denotes a straight or branched $C_2$ to $C_6$ alkyl; $R^4$ denotes a halogen, and Hal denotes a halogen) with a hydrosulfide.

7. A process for the production of a 3(2H)-pyridazinone derivative, as claimed Claim 6, wherein the product of interaction is acidified to convert it into its free acid form.